# EUROPEAN PATENT SPECIFICATION

(11) **EP 3 133 941 B1**
(45) Date of publication and mention of the grant of the patent: **01.07.2020**
(21) Application number: 15711442.2
(22) Date of filing: 10.03.2015
(51) Int. Cl.: A61P 3/00, A61P 3/04, A61P 25/02, A23L 33/00, A23L 33/16, A23L 33/105

(54) **COMPOSITION FOR USING CINNAMALDEHYDE AND ZINC FOR WEIGHT MANAGEMENT**
ZUSAMMENSETZUNG ZUR VERWENDUNG VON ZIMTALDEHYD UND ZINK ZUR GEWICHTSVERWALTUNG
COMPOSITION UTILISANT DU CINNAMALDÉHYDE ET DU ZINC POUR LA GESTION DU POIDS

(30) Priority: 20.03.2014 US 201461968096 P
(43) Date of publication of application: 01.03.2017
(73) Proprietor: Société des Produits Nestlé S.A., 1800 Vevey (CH)
(72) Inventor: MICHLIG GONZALEZ, Stéphanie, CH-1052 Le Mont-sur-Lausanne (CH); MEYLAN MERLINI, Jenny, CH-1018 Lausanne (CH); BURBIDGE, Adam, CH-1273 Arzier (CH); LE COUTRE, Johannes, CH-1009 Pully (CH)
(74) Representative: Stephen, Paula-Marie
(86) International application number: PCT/EP2015/054916
(87) International publication number: WO 2015/140002

(56) References cited:
- DE-U1-202004 017 113
- C Handramita Bora: "Effects of Cinnamon on Weight Loss", Buzzle, 21 November 2012 (2012-11-21), pages 1-3, XP055186730, Retrieved from the Internet: URL:http://www.buzzle.com/articles/cinnamo n-weight-loss-effects.html [retrieved on 2015-04-29]
- Joe Martino: "Honey & Cinnamon - The Natural Cures & Health Benefits", , 23 February 2014 (2014-02-23), pages 1-7, XP055186627, Retrieved from the Internet: URL:http://www.collective-evolution.com/20 14/02/23/honey-cinnamon-natural-cures-heal th-benefits/ [retrieved on 2015-04-29]
- DATABASE WPI Week 201263 Thomson Scientific, London, GB; AN 2011-P50685 XP002739088, "5' adenosine monophosphate - activated protein kinase activator for preparing e.g. pharmaceutical composition, to treat and prevent e.g. obesity, comprises cinnamic aldehyde as active ingredient", & KR 2011 0123002 A (UNIV KYUNGHEE IND COOP) 14 November 2011 (2011-11-14)
- TOMOKO KOMATSU ET AL: "Primary alcohols activate human TRPA1 channel in a carbon chain length-dependent manner", PFLÜGERS ARCHIV - EUROPEAN JOURNAL OF PHYSIOLOGY, SPRINGER, BERLIN, DE, vol. 463, no. 4, 6 January 2012 (2012-01-06), pages 549-559, XP035026248, ISSN: 1432-2013, DOI: 10.1007/S00424-011-1069-4

## Description

### BACKGROUND

The present disclosure generally relates to methods and compositions for weight management. More specifically, the present disclosure relates to administering an amount of cinnamaldehyde that is suitable for oral consumption and, in combination with zinc, increases energy expenditure and fat oxidation.

During the past decades, the prevalence of obesity has increased worldwide to epidemic proportion. Approximately 1 billion of people worldwide are overweight or obese, conditions that increase mortality, mobility and economical costs. Obesity develops when energy intake is greater than energy expenditure, the excess energy being stored mainly as fat in adipose tissue. Body weight loss and prevention of weight gain can be achieved by reducing energy intake or bioavailability, increasing energy expenditure, and/or reducing storage as fat.

Research on the molecular mechanisms underlying pungent sensations revealed the existence of two cation channels, TRPV1 (transient receptor potential VI) and TRPA1 (transient receptor potential A1) that are expressed in the somatosensory fibers innervating the oral cavity. TRPV1 is the receptor for heat and burning sensations such as capsaicin, the spicy compound of chili peppers. TRPA1 responds to cold and pungent compounds; at moderate concentrations, TRPA1 agonists exhibit a pleasant tingling sensation.

The TRPV1 agonist capsaicin is well known as increasing energy expenditure and fat oxidation, but the efficient doses are intermediate to high (20 mg and more). See, e.g., Ludy et al, "The effects of hedonically acceptable red pepper doses on thermogenesis and appetite," Physiol. Behav., Mar. 1, 102(3-4): 251-8 (2011). Moreover, capsaicin is a particularly pungent and toxic compound. Physiological effects associated with oral administration of capsaicin include a burning sensation of heat from the mid-tongue to the throat, shortness of breath, fainting, nausea, and spontaneous vomiting. As a result, only small quantities of capsaicin may be administered without causing discomfort to the individual. Food products containing capsaicin are frequently not accepted by the consumer because such products provide a very unpleasant mouth feeling. In particular, the burning effects are considered to be very unsavory, affecting the consumption of the food product.

Capsaicin has been shown to have an impact on human metabolism. Whereas,for example, a study that investigated the effect of mustard, horseradish, black pepper and ginger on energy balance and food intake in humans did not identify any effect of these raw spices. Gregersen et al., "Acute effects of mustard, horseradish, black pepper and ginger on energy expenditure, appetite, ad libitum energy intake and energy balance in human subjects," Br. J. Nutr., 5:1-8 (July 2012). However, the effective dosage of capsaicin is too intense to be included in a food product, due to spicy taste, or to be ingested, due to gastrointestinal intolerance.

C Handramita Bora, Buzzle, 21 November 2012, pages 1-3 (XP055186730) and Joe Martino, 23 February 2014, pages 1-7 (XP055186627) describe prepartion containing cinnamon and honey, and state that honey and cinnamon can aid in weight loss.

The cinnamon-derived compound cinnamaldehyde is a α,β-unsaturated aldehyde that activates TRPA1, but not TRPV1 or TRPM8, with an EC50 of approximately 10 µM. Cinnamaldehyde interacts with TRPA1 in a covalent manner. Cinnamaldehyde has a flavor that is less intense than capsaicin. Nevertheless, cinnamaldehyde is pungent at relatively high concentrations and has a strong cinnamon flavor.

### SUMMARY

The present inventors surprisingly and unexpectedly identified a synergy of cinnamaldehyde and zinc on the pharmacological activity of TRPA1. Using this synergy, the effective amount of cinnamaldehyde can be decreased by supplementing the cinnamaldehyde with small amount of zinc. The decreased amount of cinnamaldehyde can reduce the aromatic impact while maintaining a good efficacy on the activity of TRPA1. Moreover, the synergy only requires a low concentration of zinc (<1 µM), which is advantageous because individuals may already receive zinc through their diet, especially if supplements are consumed.

The present inventors identified a synergy of cinnamaldehyde and zinc on the pharmacological activity of TRPA1 expressed in a cellular model. To the best knowledge of the inventors, this is the first time that the synergy of this combination has been shown. This synergy is significant because cinnamaldehyde obtains a significantly higher impact on energy expenditure and sympathetic nervous system activity and an equivalent effect on fat oxidation compared to capsaicin, at a flavoring level of cinnamaldehyde judged significantly less intense than capsaicin.

Accordingly, there is provided a composition for use according to claim 1.

In an embodiment, the composition comprises cinnamon essential oil extract that provides at least a portion of the cinnamaldehyde.

In an embodiment, at least a portion of the cinnamaldehyde is selected from the group consisting of isolated cinnamaldehyde and synthesized cinnamaldehyde.

In an embodiment, the composition is a food product in which the cinnamaldehyde is present at flavouring concentration from 31.87 ppm (condiments, relishes) up to 6191 ppm (chewing gum) (Fenaroli's Handbook; Burdock, 2010).

In an embodiment, the composition is a food product in which the cinnamaldehyde:zinc ratio is 1:0.5 to 1:0.005, preferably 1:0.03 (in molarity).

In another embodiment, the present disclosure provides a composition for promoting weight losswhich the composition comprising cinnamaldehyde and zinc according to claim 1 is administerd to an individual on a weight loss program.

In an embodiment, the weight loss program is selected from the group consisting of a low-fat diet, a low-carbohydrate diet, a low-calorie diet, a very low-calorie diet, endurance training, strength training, and combinations thereof.

In another embodiment, the present disclosure provides a method for making a food product for use in promoting weight loss. The method comprises adding cinnamaldehyde and zinc to a component selected from the group consisting of protein, carbohydrate, fat and combinations thereof, wherein the cinnamaldehyde is present at a flavouring concentration from 31.87 ppm to 6191 ppm and the cinnamaldehyde:zinc ratio is 1:0.5 to 1:0.005 (in molarity) .

An advantage of the present disclosure is to increase energy expenditure.

Another advantage of the present disclosure is to increase sympathetic nervous system activity.

Still another advantage of the present disclosure is to increase fat oxidation.

Yet another advantage of the present disclosure is to increase energy expenditure, sympathetic nervous system activity, and fat oxidation with a compound that can be easily and safely used in food products.

An additional advantage of the present disclosure is to increase energy expenditure, sympathetic nervous system activity, and fat oxidation with a naturally-occurring compound that can be found in spices.

Another advantage of the present disclosure is to increase energy expenditure, sympathetic nervous system activity, and fat oxidation with tolerable side effects or no side effects.

Yet another advantage of the present disclosure is to support weight management, promote weight loss, and/or treat or prevent obesity or overweight.

Still another advantage of the present disclosure is to increase energy expenditure, sympathetic nervous system activity, and fat oxidation with a compound that has increased acceptability, reduced pungency, and improved tolerance in the gastrointestinal tract relative to capsaicin.

Another advantage of the present disclosure is to supplement cinnamaldehyde with zinc so that less cinnamaldehyde is required to increase energy expenditure.

Additional features and advantages are described herein, and will be apparent from, the following Detailed Description and the Figures.

### BRIEF DESCRIPTION OF THE FIGURES

Fig. 1 shows the chemical structure of cinnamaldehyde.
Fig. 2 shows a proposed mechanism by which cinnamaldehyde can increase energy expenditure, sympathetic nervous system activity, and fat oxidation.
Fig. 3 shows a graph of energy expenditure as a function of time elapsed after ingestion of various compounds.
Fig. 4 shows a graph of energy expenditure based on AUC after ingestion of various compounds.
Fig. 5 shows a graph of postprandial fat oxidation as a function of time elapsed after ingestion of various compounds.
Fig. 6 shows a graph of postprandial fat oxidation based on AUC after ingestion of various compounds.
Fig. 7 shows a graph of nose temperature increases experienced after ingestion of various compounds.
Fig. 8 shows a graph of chin temperature, relative to baseline, as a function of time elapsed after ingestion of various compounds.
Fig. 9 shows taste testing results comparing 4.88 ppm of capsaicin and 350 ppm of cinnamaldehyde.
Fig. 10 shows a graph of *in vitro* measurement of the activity of TRP channels expressed in CHO cells by measuring the intracellular calcium concentration with a fluorescent dye.

### DETAILED DESCRIPTION

All percentages expressed herein are by weight of the total weight of the composition unless expressed otherwise. When reference is made to the pH, values correspond to pH measured at 25°C with standard equipment. As used in this disclosure and the appended claims, the singular forms "a," "an" and "the" include plural referents unless the context clearly dictates otherwise. As used herein, "about" is understood to refer to numbers in a range of numerals, for example the range of -10% to +10% of the referenced number. Moreover, all numerical ranges herein should be understood to include all integers, whole or fractions, within the range. The compositions disclosed herein may lack any element that is not specifically disclosed herein. Thus, a disclosure of an embodiment using the term "comprising" includes a disclosure of embodiments "consisting essentially of' and "consisting of' the components identified.

"Prevention" includes reduction of risk and/or severity of a condition or disorder. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition. A treatment can be patient- or doctor-related.

As used herein, an "effective amount" is an amount that prevents a deficiency, treats a disease or medical condition in an individual or, more generally, reduces symptoms, manages progression of the diseases or provides a nutritional, physiological, or medical benefit to the individual.

"Animal" includes, but is not limited to, mammals, which includes but is not limited to, rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage. As used herein, the term "patient" is understood to include an animal, especially a mammal, and more especially a human that is receiving or intended to receive treatment, as treatment is herein defined. While the terms "individual" and "patient" are often used herein to refer to a human, the present disclosure is not so limited. Accordingly, the terms "individual" and "patient" refer to any animal, mammal or human that can benefit from the treatment.

"Overweight" is defined for a human as a BMI between 25 and 30. "Obese" is defined for a human as a BMI greater than 30. "Weight loss" is a reduction of the total body weight. Weight loss may, for example, refer to the loss of total body mass in an effort to improve fitness, health, and/or appearance. "Weight management" or "weight maintenance" relates to maintaining a total body weight. For example, weight management may relate to maintaining a BMI in the area of 18.5-25 which is considered to be normal.

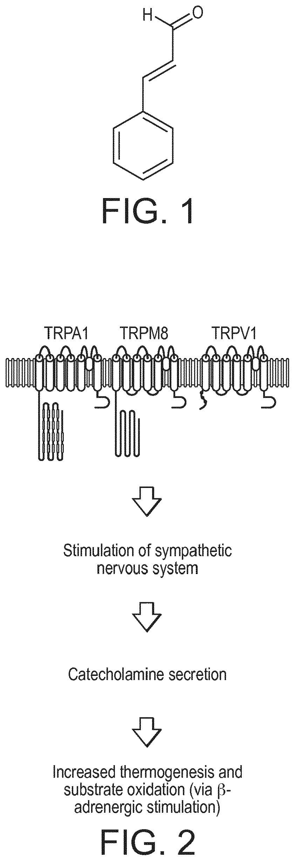

As set forth above, the present inventors surprisingly and unexpectedly found a synergy of cinnamaldehyde and zinc on the pharmacological activity of TRPA1. Using this synergy, the effective amount of cinnamaldehyde can be decreased by supplementing the cinnamaldehyde with small amount of zinc. Consequently, unlike cinnamaldehyde in the absence of zinc, the combination of cinnamaldehyde and zinc can impact energy expenditure, sympathetic nervous system activity, and fat oxidation at concentrations in food that are safe and tolerable both in flavor/taste and in the gastrointestinal tract. Moreover, the synergy only requires a low concentration of zinc (*in vitro* <1 µM). Without being bound by theory, the inventors believe that cinnamaldehyde and zinc synergistically stimulate the sympathetic nervous system and, as a result, catecholamine secretion. The increased catecholamine secretion enhances thermogenesis and substrate oxidation by β-adrenergic stimulation. See Fig. 2.

Accordingly, the composition provided by the present disclosure comprises an amount of the cinnamaldehyde that is safe and orally tolerable, for example does not cause an unpleasant mouth feeling, and, in combination with the zinc, also effective to increase at least one of energy expenditure, sympathetic nervous system activity, or fat oxidation, relative to an otherwise identical composition lacking cinnamaldehyde and zinc.

Cinnamaldehyde is available commercially. The cinnamaldehyde in the composition can be provided in a cinnamon essential oil extract, for example an extract from steam distillation of the oil of cinnamon bark; can be isolated cinnamaldehyde, for example isolated from cinnamon essential oil; or can be synthesized cinnamaldehyde, for example the product of aldol condensation of benzaldehyde and acetaldehyde. The concentration of cinnamaldehyde in the composition is at flavouring concentration from 31.87 ppm (condiments, relishes) up to 6191 ppm (chewing gum) (Fenaroli's Handbook; Burdock, 2010). In an embodiment, the cinnamaldehyde is present in composition in an amount of about 100.0 ppm or less.

As non limiting examples, the cinnamaldehyde can be present in the following compositions as follows:
alcoholic beverage: up to 498.8 ppm, such as about 435.6 ppm
baked good: up to 367.4 ppm, such as about 273.8 ppm
chewing gum: up to 6191.0 ppm, such as about 1533.0 ppm
condiment or relish: up to 31.87 ppm, such as about 17.48 ppm
frozen dairy product: up to 77.96 ppm, such as about 72.98 ppm
fruit ice: up to 900.0 ppm, such as 900.0 ppm
gelatin or pudding: up to 109.4 ppm, such as about 100.3 ppm
gravy: up to 800.0 ppm, such as about 640.0 ppm
hard candy: up to 1003.0 ppm, such as about 792.2 ppm
meat product: up to 39.09 ppm, such as about 6.97 ppm
non-alcoholic beverage: up to 67.82 ppm, such as about 52.71 ppm
soft candy: up to 370.0 ppm, such as 370.0 ppm

Preferred forms of zinc include zinc chloride, zinc sulfate, zinc lactate and zinc citrate. The cinnamaldehyde:zinc ratio is 1:0.5 to 1:0.005, preferably 1:0.03 (in molarity).

In an embodiment, the composition comprising cinnamaldehyde and zinc can be used in a method to support weight management or promote weight loss. For example, the composition can be administered to an individual, such as a mammal, that is managing their weight or undergoing a weight loss program. The weight loss program may include, for example, a weight loss diet (e.g., one or more of a low-fat diet, for example a diet with less than 20% of the calories from fat, preferably less than 15% from fat; a low-carbohydrate diet, for example a diet with less than 20% of the calories from carbohydrates; a low-calorie diet, for example a diet with less calories per day relative to the individual's previous intake before the diet, or a diet with less calories per day relative to an average person of similar body type; or a very low-calorie diet, for example a diet with 800 kcal (3,300 kJ) per day or less). Additionally or alternatively, the weight loss program may include a weight loss training regimen (e.g. endurance and/or strength training). In another embodiment, the composition comprising cinnamaldehyde and zinc can be used in a method for preventing obesity or overweight by administering the composition to an individual at risk thereof. In yet another embodiment, the composition comprising cinnamaldehyde and zinc can be used in a method for treating obesity or overweight by administering the composition to an individual in need thereof. In an embodiment, the composition comprising cinnamaldehyde and zinc is administered to a mammal, such as a human. The composition can also comprise an additional weight loss ingredient.

The composition comprising cinnamaldehyde and zinc may be a medicament, a food product, a medical food, an oral nutritional supplement, a nutritional composition, an oral cosmetics or a supplement to a food product and is preferably orally administered. A medical food product is specially formulated and intended for the dietary management of diseases or medical conditions (e.g., prevent or treat diseases or undesirable medical conditions). A medical food product can provide clinical nutrition, for example fulfilling special nutritional needs of patients with a medical condition or other persons with specific nutritional needs. A medical food product can be in the form of a complete meal, part of a meal, as a food additive, or a powder for dissolution.

A food product, medical food or nutritional composition includes any number of optional additional ingredients, including conventional food additives, for example one or more proteins, carbohydrates, fats, acidulants, thickeners, buffers or agents for pH adjustment, chelating agents, colorants, emulsifiers, excipients, flavor agents, minerals, osmotic agents, a pharmaceutically acceptable carrier, preservatives, stabilizers, sugars, sweeteners, texturizers and/or vitamins. The optional ingredients can be added in any suitable amount.

A food product, medical food or nutritional composition can be in any oral nutritional form, e.g. as a health drink, as a ready-made drink, optionally as a soft drink, including juices, milk-shake, yogurt drink, smoothie or soy-based drink, in a bar, or dispersed in foods of any sort, such as baked products, cereal bars, dairy bars, snack-foods, soups, breakfast cereals, muesli, candies, tabs, cookies, biscuits, crackers (such as a rice crackers), and dairy products.

A supplement may be in the form of tablets, capsules, pastilles or a liquid, for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins or the like), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like.

The supplement can be added in a product acceptable to the consumer as an ingestible carrier or support. Non-limiting examples of such carriers or supports are a pharmaceutical, a food composition, and a pet food composition. Non-limiting examples for food and pet food compositions are milks, yogurts, curds, cheeses, fermented milks, milk-based fermented products, fermented cereal based products, milk-based powders, human milks, preterm formulas, infant formulas, oral supplements, and tube feedings.

### EXAMPLES

The following non-limiting examples present scientific data developing and supporting the concept of administering the combination of cinnamaldehyde and zinc to synergistically activate TRPA1 to increase at least one of energy expenditure, sympathetic nervous system activity, or fat oxidation, without imparting an intolerable taste or gastrointestinal effect.

### Example 1

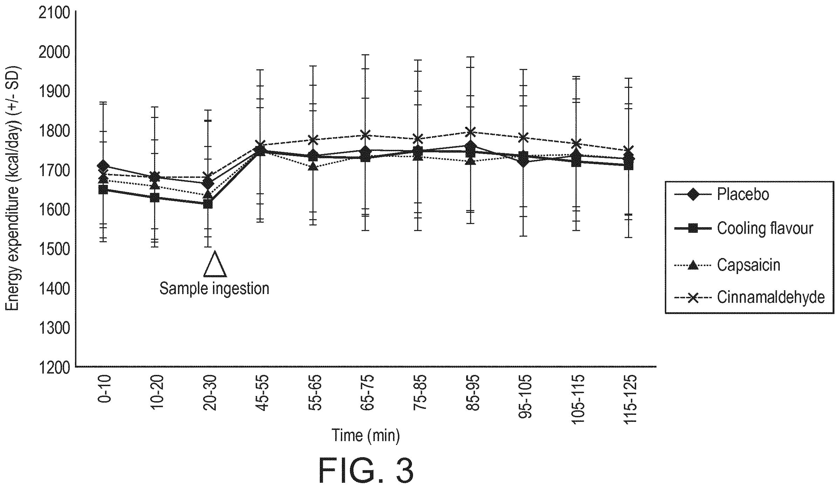

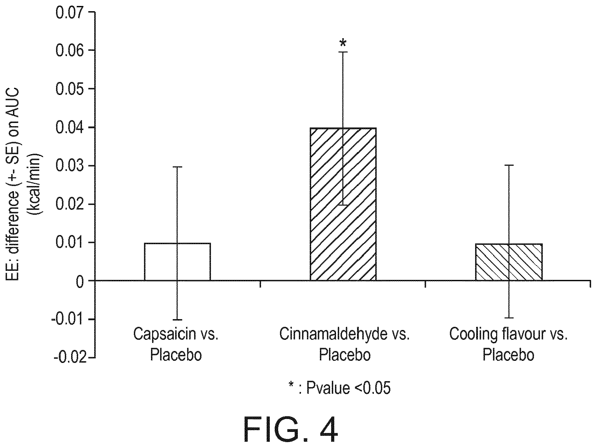

Human subjects were administered placebo, a cooling flavor, capsaicin, or cinnamaldehyde. The energy expenditure was measured over the eighty minutes following ingestion. Fig. 3 shows a graph of energy expenditure as a function of time elapsed after ingestion of the various compounds. Fig. 4 shows a graph of energy expenditure based on AUC after ingestion of the various compounds. Figs. 3 and 4 demonstrate that energy expenditure is increased after cinnamaldehyde ingestion compared to placebo.

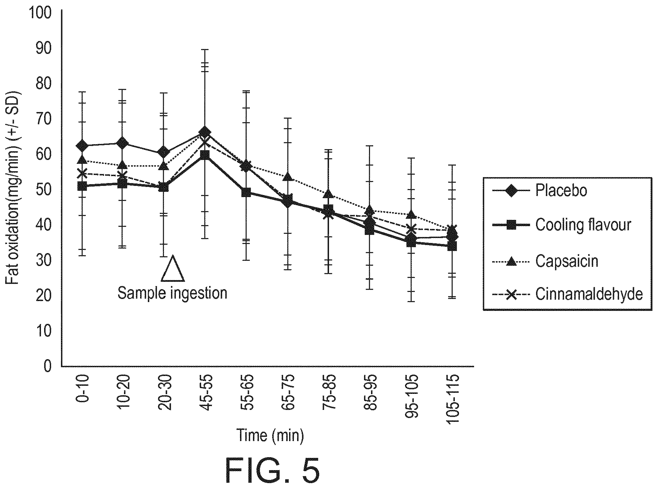

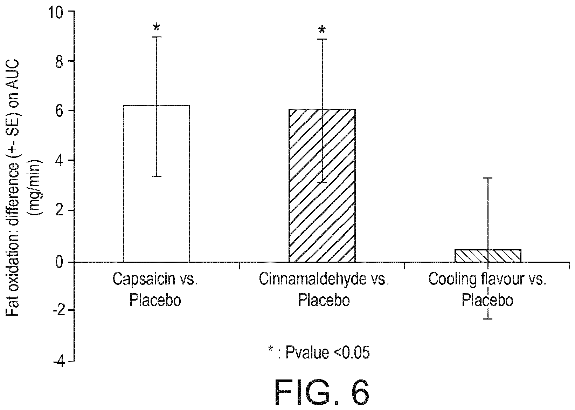

The postprandial fat oxidation was measured over the 90 minutes following ingestion of the various compounds. Fig. 5 shows a graph of postprandial fat oxidation as a function of time elapsed after ingestion of the various compounds. Fig. 6 shows a graph of postprandial fat oxidation based on AUC after ingestion of various compounds. Figs. 5 and 6 demonstrate that postprandial fat oxidation is maintained at higher levels after cinnamaldehyde ingestion compared to placebo.

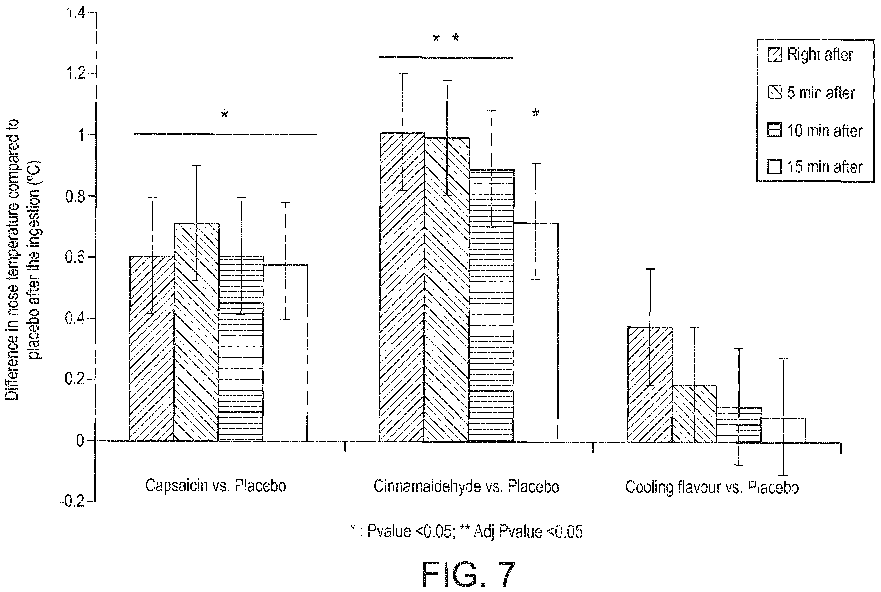

The nose temperature of the subjects was analyzed over the fifteen minutes following ingestion of the various compounds. Fig. 7 shows a graph of the nose temperature increases that were experienced after ingestion of the various compounds. Fig. 7 demonstrates that capsaicin and cinnamaldehyde increase nose temperatures for the fifteen minutes following ingestion, suggesting stimulation of the same autonomic thermoregulation pathway.

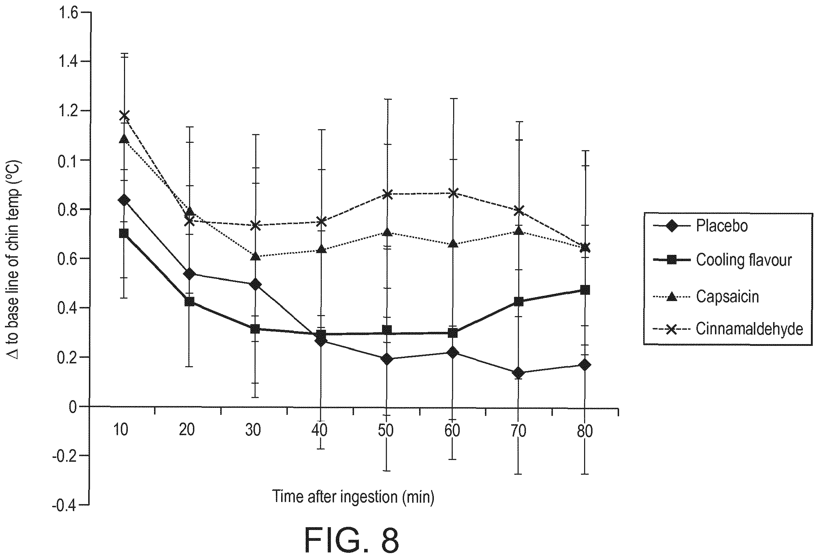

The chin temperature of the subjects was measured over the eighty minutes following ingestion of the various compounds. Fig. 8 shows a graph of the chin temperature, relative to baseline, as a function of time elapsed after ingestion of the various compounds Fig. 8 demonstrates that cinnamaldehyde increases chin temperature for a prolonged time after ingestion, indicating increased blood flow, probably reflecting sympathetic autonomic activity. The differences in chin temperature relative to placebo are provided in Table 1.

These results indicate that capsaicin and cinnamaldehyde might induce the same short term autonomic thermoregulation response by inducing a vasodilator reflex on the capillary of the nose. The increased sympathetic activity identified by measuring the facial temperature (increased blood flow on the chin) might explain the increased energy expenditure measured by indirect calorimetry.

### AUC

**Table 1**

| **Comparisons** | **Difference** | **SE** | **98.33%Cl** | **Raw p-value** | **Adjusted p-value** |
|---|---|---|---|---|---|
| Z10 Cap vs PI | 25,26 | 13.943 | [-9.583;60.104] | 0.0775 | 0.2325 |
| Z10 Cin vs PI | 34.072 | 13.567 | [0.166;67.977] | 0.0162 | **0.0486** |
| Z10 CF vs PI | 3.486 | 13.557 | [-30.394;37.367] | 0.7984 | 1 |

### Example 2

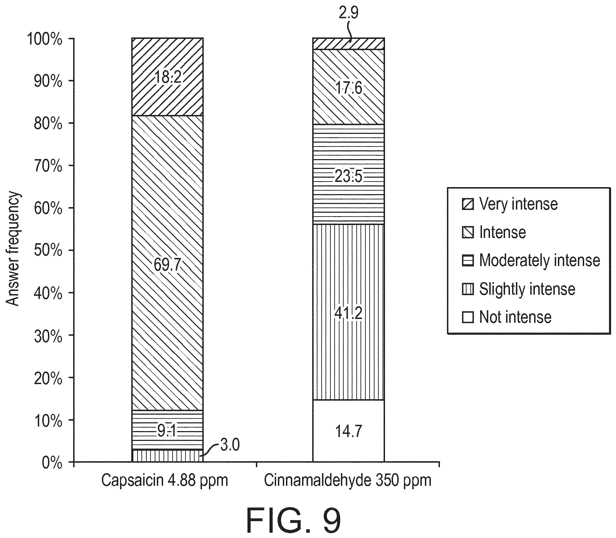

Human subjects were administered a composition comprising 4.8 ppm capsaicin or 350 ppm cinnamaldehyde. Fig. 9 shows a graph of the comparative taste testing results. 87.9% of the participants judged capsaicin intense to very intense, compared to only 20.5% for cinnamaldehyde. To achieve a similar effect on fat oxidation of capsaicin and cinnamaldehyde, the dose of capsaicin is about 1.5 times less than the maximum that can be used as a flavor (7 ppm according to Fenaroli's Handbook; Burdock, 2010), and the dose of cinnamaldehyde is about 17.5 times less than the maximum that can be used as a flavor (6191.0 ppm according to Fenaroli's Handbook; Burdock, 2010).

### Example 3

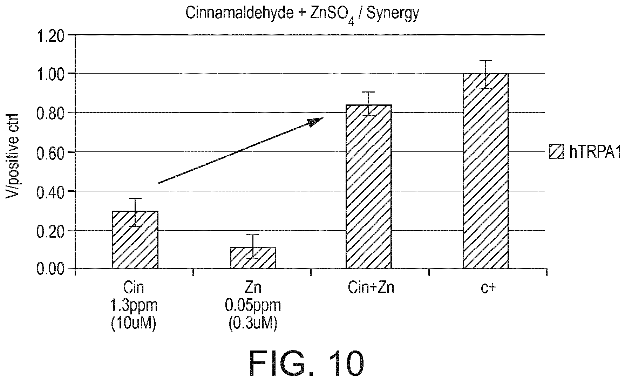

The *in vitro* activity of hTRPA1 expressed in CHO cells was measured for 10 µM cinnamaldehyde and 0.3 µM zinc individually, as well as the combination. The results are shown in Fig. 10 and show a synergistic effect when cinnamaldehyde and zinc are combined (Cin+Zinc). C+ represents the experimental positive control for this test and is cinnamaldehyde at 50 mM which give a maximum efficacy according to a dose-response curve.

## Claims

1. A composition comprising cinnamaldehyde and zinc for use in preventing obesity or overweight in an individual at risk thereof, treating obesity in an obese individual, and/or promoting weight loss or weight maintenance in an individual in need thereof, wherein the concentration of cinnamaldehyde in the composition is at flavouring concentration from 31.87 ppm up to 6191 ppm and the cinnamaldehyde:zinc ratio is 1:0.5 to 1:0.005 in molarity.

2. The composition for use according to Claim 1 wherein the composition comprises cinnamon essential oil extract that provides at least a portion of the cinnamaldehyde.

3. The composition for use according to Claim 1 wherein at least a portion of the cinnamaldehyde is selected from the group consisting of isolated cinnamaldehyde and synthesized cinnamaldehyde.

4. The composition for use according to claim 1 wherein the cinnamaldehyde is present in composition in an amount of about 100.0 ppm or less.

5. The composition for use according to claim 1 wherein the cinnamaldehyde:zinc ratio is 1:0.5 to 1:0.03 in molarity.

6. A composition for use in promoting weight loss according to claim 1 wherein the composition is administered to an individual on a weight loss program.

7. The composition for use according to Claim 6 wherein the weight loss program is selected from the group consisting of a low-fat diet, a low-carbohydrate diet, a low-calorie diet, a very low-calorie diet, endurance training, strength training, and combinations thereof.

8. A method for making a food product for weight loss, the method comprising adding cinnamaldehyde and zinc to a component selected from the group consisting of protein, carbohydrate, fat and combinations thereof, wherein the cinnamaldehyde is present at a flavouring concentration from 31.87 ppm to 6191 ppm and the cinnamaldehyde:zinc ratio is 1:0.5 to 1:0.005 in molarity.

## Patentansprüche

1. Zusammensetzung, umfassend Zimtaldehyd und Zink zur Verwendung bei der Vorbeugung von Fettleibigkeit oder Übergewicht in einem Individuum, das gefährdet ist, daran zu erkranken, der Behandlung von Fettleibigkeit in einem fettleibigen Individuum und/oder der Förderung von Gewichtsverlust oder Gewichtsverwaltung in einem Individuum, das einer solchen Behandlung bedarf, wobei die Konzentration von Zimtaldehyd in der Zusammensetzung in einer Aromastoffkonzentration von 31,87 ppm bis zu 6191 ppm vorliegt und das Zimtaldehyd : Zink-Verhältnis in der Molarität 1 :0,5 bis 1 : 0,005 beträgt.

2. Zusammensetzung zur Verwendung nach Anspruch 1, wobei die Zusammensetzung Extrakt aus ätherischem Öl von Zimt umfasst, der zumindest einen Teil des Zimtaldehyds bereitstellt.

3. Zusammensetzung zur Verwendung nach Anspruch 1, wobei zumindest ein Teil des Zimtaldehyds ausgewählt ist aus der Gruppe bestehend aus isoliertem Zimtaldehyd und synthetisiertem Zimtaldehyd.

4. Zusammensetzung zur Verwendung nach Anspruch 1, wobei das Zimtaldehyd in der Zusammensetzung in einer Menge von etwa 100,0 ppm oder weniger vorhanden ist.

5. Zusammensetzung zur Verwendung nach Anspruch 1, wobei das Zimtaldehyd : Zink-Verhältnis in der Molarität 1 :0,5 bis 1 :0,03 beträgt.

6. Zusammensetzung zur Verwendung bei der Förderung des Gewichtsverlustes nach Anspruch 1, wobei die Zusammensetzung einem Individuum in einem Gewichtsverlustprogramm verabreicht wird.

7. Zusammensetzung zur Verwendung nach Anspruch 6, wobei das Gewichtsverlustprogramm ausgewählt ist aus der Gruppe bestehend aus einer fettarmen Diät, einer kohlenhydratarmen Diät, einer kalorienarmen Diät, einer sehr kalorienarmen Diät, einem Ausdauertraining, einem Krafttraining und Kombinationen davon.

8. Verfahren zur Herstellung eines Nahrungsmittelprodukts für Gewichtsverlust, das Verfahren umfassend die Zugabe von Zimtaldehyd und Zink zu einer Komponente, ausgewählt aus der Gruppe bestehend aus Protein, Kohlenhydrat, Fett und Kombinationen davon, wobei das Zimtaldehyd in einer Aromastoffkonzentration von 31,87 ppm bis 6191 ppm vorhanden ist und das Zimtaldehyd : Zink-Verhältnis in der Molarität 1 : 0,5 bis 1 : 0,005 beträgt.

## Revendications

1. Composition comprenant du cinnamaldéhyde et du zinc utilisable pour prévenir l'obésité ou le surpoids chez un sujet à risque, traiter l'obésité chez un sujet obèse, et/ou favoriser la perte de poids ou le maintien du poids chez un sujet qui en a besoin, dans laquelle la concentration de cinnamaldéhyde dans la composition est à une concentration aromatisante allant de 31,87 ppm jusqu'à 6191 ppm et le rapport cinnamaldéhyde:zinc va de 1:0,5 à 1:0,005 en molarité.

2. Composition utilisable selon la revendication 1 dans laquelle la composition comprend de l'extrait d'huile essentielle de cannelle qui fournit au moins une partie du cinnamaldéhyde.

3. Composition utilisable selon la revendication 1 dans laquelle au moins une partie du cinnamaldéhyde est choisie dans le groupe constitué de cinnamaldéhyde isolé et cinnamaldéhyde synthétisé.

4. Composition utilisable selon la revendication 1 dans laquelle le cinnamaldéhyde est présent dans la composition en une quantité d'environ 100,0 ppm ou moins.

5. Composition utilisable selon la revendication 1 dans laquelle le rapport cinnamaldéhyde:zinc va de 1:0,5 à 1:0,03 en molarité.

6. Composition utilisable pour favoriser une perte de poids selon la revendication 1 dans laquelle la composition est administrée à un sujet selon un programme de perte de poids.

7. Composition utilisable selon la revendication 6 dans laquelle le programme de perte de poids est choisi dans le groupe constitué d'un régime pauvre en matières grasses, un régime pauvre en glucides, un régime faiblement calorique, un régime très faiblement calorique, un entraînement d'endurance, de la musculation, et des combinaisons de ceux-ci.

8. Procédé de fabrication d'un produit alimentaire pour perte de poids, le procédé comprenant l'ajout de cinnamaldéhyde et de zinc à un composant choisi dans le groupe constitué de protéine, glucide, matière grasse et des combinaisons de ceux-ci, dans lequel le cinnamaldéhyde est présent à une concentration aromatisante allant de 31,87 ppm à 6191 ppm et le rapport cinnamaldéhyde:zinc va de 1:0,5 à 1:0,005 en molarité.
